# EUROPEAN PATENT APPLICATION

(11) **EP 0 539 336 A1**
(43) Date of publication of application: **28.04.1993**
(21) Application number: 92830564.8
(22) Date of filing: 12.10.1992
(51) Int. Cl.: A61K 31/205, A61K 31/22

(54) **Use of L-carnitine or of acyl L-carnitine for the treatment of idiopathic oligoasthenospermias**

(30) Priority: 21.10.1991 IT RM910796
(71) Applicant: Sigma-Tau Industrie Farmaceutiche Riunite S.p.A., I-00144 Roma (IT)
(72) Inventor: Cavazza, Claudio, I-00186 Roma (IT)
(74) Representative: Fassi, Aldo

(57) **Abstract**

A novel therapeutical use of L-carnitine and acyl L-carnitines wherein the acyl is a straight or branched-chain acyl group having 2-6 carbon atoms and pharmacologically acceptable salts thereof for treating idiopathic oligoasthenospermias is disclosed.

## Description

The present invention relates to a new therapeutic use of L-carnitine, some acyl L-carnitines and the pharmacologically acceptable salts thereof for the treatment of idiopathic oligoasthenospermias.

The acyl L-carnitines useful for the new therapeutical use of the present invention are those wherein the acyl group is a straight or branched-chain group having from 2 to 6 carbon atoms.

Particularly preferred are acetyl, propionlyl, butyryl, valeryl and isovaleryl L-carnitines.

Pharmaceutically acceptable salts of L-carnitine include all pharmaceutically acceptable salts which are prepared by the addition of acid to L-carnitine, and which do not give rise to undesirable toxic or collateral effects. The formation of pharmaceutically acceptable acid addition salts is well known in pharmaceutical technology. Non-limiting examples of suitable salts include the chloride, bromide, orotate, acid aspartate, acid citrate, acid phosphate, fumarate, acid fumarate, lactate, maleate, acid maleate, acid oxalate, acid sulfate, glucose phosphate, tartrate and acid tartrate salts. Other suitably acceptable salts which are nontoxic and provide substantially similar results to administration of L-carnitine and the above-identified pharmaceutical salts will be readily apparent to one having ordinary skill in the art and are considered to be equivalent to the salts enumerated above.

For the sake of simplicity and clarity, herinbelow reference will be made to L-carnitine only, it being understood, however, that whatever disclosed in connection with L-carnitine equally applies to the above identified acyl L-carntines and pharmacologically acceptable salts thereof.

Previous therapeutical uses of L-carnitine are already known. For instance, L-carnitine has been used in the cardiovascular field in the treatment of acute and chronic myocardial ischaemia, angina pectoris, cardiac arrhythmias and insufficiency. In nephrology, L-carnitine has been administered to chronic uraemic patients who are subjected to regular haemodialysis treatment with a view to counteracting muscular asthenia and the onset of muscular cramps. Further therapeutical uses are the restoration of the HDL/LDL+VLDL ratio to normal and in total parenteral nutrition. The use of L-carnitine for the treatment of certain myopathies and muscular dystrophies is also known.

It is, therefore, unexpected and surprising the therapeutic efficacy of the oral or parenteral administration of L- carnitine to patients suffering from idiopathic oligoasthenospermia insofar as these patients usually exhibit absolutely normal serum and tissue carnitine levels.

A study was conducted in order to assess the alterations induced by L-carntine administration on spermatozoa motility. The motility was determined both by the conventional assay and the computerized method in patients suffering from idiopathic sperm hypomotility.

100 patients suffering from idiopathic sperm hypomotility were treated for 4 months with L-carnitine.

The test data showed that L-carnitine significantly stimulates spermatozoa motility both qualitatively and quantitatively and more precisely brings about an increase in the percentage of fast motile spermatozoa as well as an increase in spermatozoa forward motility.

Although the daily dose to the administered depends on the age, weight and general condition of the subject, utilizing sound professional judgement, it has been found that, generally, from about 10 to 30 mg of L-carnitine/kg of body weight/day or an equivalent amount of a pharmacologically acceptable salt thereof, is a suitable dose.

L-carnitine is compounded into the pharmaceutical compositions by using the usual excipients, diluents and adjuvant agents which are well-known in pharmaceutical technology for preparing orally and parenterally administrable compositions.

It has also been found that a pharmaceutical composition in unit dosage form which is particularly suited for the foregoing therapeutical uses comprises from about 500 to about 1,000 mg of L-carnitine.

## Claims

1. Use of L-carnitine and acyl L-carnitine wherein the acyl group is a straight or branched-chain group having 2-6 carbon atoms, and the pharmacologically acceptable salts thereof for the therapeutical treatment of idiopathic oligoasthenospermias.

2. Use of L-carnitine and acyl L-carnitine wherein the acyl group is a straight or branched-chain group having 2-6 carbon stoms, and the pharmacologically acceptable salts thereof for producing a medicament for the therapeutical treatment of idiopathic oligoasthenospermias.

3. Use of L-carnitine and acyl L-carnitines according to the preceding claims which comprises orally or parenterally administering about 10-30 mg L-carnitine/kg body weight/day to a patient suffering from idiopathic oligoasthenospermia.

4. Use according to anyone of the preceding claims wherein the acyl L-carnitine is selected from acetyl, propionyl, butyryl, valeryl and isovaleryl L-carnitine.

5. An orally or parenterally administrable composition in unit dosage form for the treatment of idiopathic oligoasthenospermias, comprising from about 500 to about 1000 mg L-carnitine or an equivalent amount of an acyl L-carnitine wherein the acyl group is a straight or branched chain acyl group having 2-6 carbon atoms or a pharmacologically acceptable salt thereof and a pharmacologically acceptable excipient therefor.
